# EUROPEAN PATENT APPLICATION

(11) **EP 4 635 576 A1**
(43) Date of publication of application: **22.10.2025**
(21) Application number: 24187976.6
(22) Date of filing: 11.07.2024
(51) Int. Cl.: A61Q 5/02, A61Q 5/12, A61K 8/20, A61K 8/44, A61K 8/46, A61K 8/66

(54) **PROTEASES FOR HAIR-CARE**

(30) Priority: 16.04.2024 EP 24170549
(71) Applicant: BASF SE, 67056 Ludwigshafen am Rhein (DE)
(72) Inventor: The designation of the inventor has not yet been filed
(74) Representative: BASF IP Association

(57) **Abstract**

The present invention is directed to hair-care compositions comprising a protease. In particular, the present invention is directed to the use of proteases in shampoo compositions. Especially, the present invention is directed to the improvement of combability of hair by adding a protease to a hair treatment composition.

## Description

### Field of the invention

The present invention is directed to hair-care compositions comprising a protease. In particular, the present invention is directed to the use of proteases in shampoo compositions. Especially, the present invention is directed to the improvement of combability of hair by adding a protease to a hair treatment composition.

### Background of the invention

Hair gets disrupted over time, especially longer hair, by friction, combing, blow drying, and UV light, resulting in a rough, unpleasant hair touch as well as in an increase in tangling, leading to difficulties in combing the hair. To smoothen the hair, conditioning agents, most often polymeric siloxanes (silicones), are usually being added to hair-care compositions. These conditioning agents cover the hair and thereby smoothen rough cuticle edges of the hair. For smoothening the hair conditioning agents must remain on the surface of the hair and shall not be easily rinsed off (leave-in conditioners). While conditioning agents are widely used in hair care compositions for their ability to improve hair texture, shine, and manageability, there are several potential disadvantages associated with their use. Because conditioning agents need to be left on the hair to be effective, they can sometimes build up on the hair over time, leading to a heavy, greasy, or dull appearance. Because conditioning agents are designed to adhere strongly to the hair, they can be difficult to remove completely, even with regular shampooing. Some conditioning agents may be incompatible with certain ingredients commonly used in hair care formulations, such as certain surfactants or active ingredients. This can lead to decreased product performance or stability. In many cases, the production of synthetic conditioning agents involves hazardous chemicals. In some cases, individuals experience sensitivity or allergic reactions to certain conditioning agents, leading to symptoms such as itching, redness, or swelling. Most importantly, conditioning agents, in particular non-natural synthetic conditioning agents, like polymeric siloxanes and many cationic polymers, are often fossil-based and/or non-biodegradable and thus impose a negative impact to the environment during production and disposal.

The present invention is based on the discovery that proteases can be used to smoothen the hair surface, making the hair feel more natural, leading to a shiny appearance and improved combability, which reduces the need to add conditioning agents, in particular the need to add synthetic conditioners, particularly non-biodegradable polymeric siloxanes.

Proteases are enzymes capable of degrading proteins and have found application in various industries such as detergents for laundry and dishwashing, as well as in skincare products. However, until now, their potential application in hair-care, in particular in shampoo products, has only been sparsely explored.

In particular, the prior art does not disclose the use of proteases in a hair-care composition as an alternative to conditioning agents to improve combability of the hair.

CN106806281 discloses a shampoo composition comprising a protease, which however comprises non-biodegradable polysiloxane as hair conditioner.

US20030091526 discloses shampoo compositions comprising enzymes capable of inducing an esterification reaction on the hair surface.

### Brief summary of the invention

In one embodiment, the present invention is directed to a hair-care composition comprising
a) one or more proteases,
b) one or more surfactants, with preferably at least one of the surfactants selected from the group consisting of
   a. ionic surfactant,
   b. amphoteric surfactant, and
   c. non-ionic surfactant,
c) preferably one or more salt,
   wherein the composition is essentially devoid of alkyl dimethyl polysiloxanes, preferably, the composition is essentially devoid of polymeric siloxanes, in particular dimethyl siloxanes, preferably essentially devoid of polymeric siloxanes and quaternary ammonium compounds, in particular polyquaternium polymers, even more preferably, the composition is essentially devoid of a synthetic conditioning polymer, most preferably, the composition is essentially devoid of any synthetic conditioning agent and
   wherein the pH of the composition is pH 4.0-8.5.

In another embodiment, the present invention is directed to a method for improving the combability of hair, preferably the dry combability, comprising the steps of
i. contacting hair with a composition of the present invention under conditions suitable for the protease of the composition to exert proteolytic activity on the hair, preferably the hair surface, and
ii. rinsing-off the composition from the hair; and
iii. optionally drying the hair.

In yet another embodiment, the present invention is directed to a method for smoothening the hair surface, comprising the steps of
i. contacting hair with a composition of the present invention under conditions suitable for the protease of the composition to exert proteolytic activity on the hair, preferably the hair surface, and
ii. rinsing-off the composition from the hair; and
iii. optionally drying the hair.

### Detailed description of the invention

### Definitions

Unless otherwise noted, the terms used herein are to be understood according to conventional usage by those of ordinary skill in the relevant art.

Before describing in detail exemplary embodiments of the present invention, definitions important for understanding the present invention are given. Unless stated otherwise or apparent from the nature of the definition, the definitions apply to all compounds, methods and uses described herein.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

Throughout this application, various publications are referenced. The disclosure of all of these publications and those references cited within those publications in their entireties are hereby incorporated by reference into this application in order to more fully describe the state of the art to which this invention pertains.

It is to be understood that the term "comprising" is not limiting. For the purposes of the present invention the term "consisting of" is considered to be a preferred embodiment of the term "comprising". If hereinafter a group is defined to comprise at least a certain number of members, this is meant to also encompass a group which consists of these members only.

The term "introduction of at least two negative charges" into a particular amino acid sequence refers to the increase of the net charge of the particular amino acid sequence by at least two negative charges. Such increase of the net charge of the particular amino acid sequence by at least two negative charges is achieved by altering the amino acid sequence and can be reached by one or more amino acid sequence alterations selected from the group consisting of substitution, deletion and insertion, preferably by one or more amino acid substitutions. The increase of the net charge of the particular amino acid sequence by at least two negative charges can be achieved by removing positive charges or by introducing negative charges or by combinations thereof. The four amino acids aspartic acid (Asp, D), glutamic acid (Glu, E), lysine (Lys, K), and arginine (Arg, R) have a side chain which can be charged at neutral pH. At pH 7.0, two are negatively charged: aspartic acid (Asp, D) and glutamic acid (Glu, E) (acidic side chains), and two are positively charged: lysine (Lys, K) and arginine (Arg, R) (basic side chains). Thus, the introduction of at least two negative charges in the amino acid sequence can be reached for instance by substituting arginine by glutamic acid, substituting two non-charged leucine residues by two glutamic acid residues, by inserting two aspartic acid residues or by deleting two lysine residues. The introduction of at least two negative charges by modification of the amino acid sequence is evaluated preferably under conditions usually occurring in an enzyme composition described herein, preferably at pH 6-11, preferably at pH 7-9, more preferably at pH 7.5-8.5, further preferred at pH 7.0-8.0, most preferably at pH 7.0 or pH 8.0.

"Parent sequence" (also called "parent enzyme" or "parent protein") is the starting sequences for introduction of changes (e.g. by introducing one or more amino acid substitutions) of the sequence resulting in "variants" of the parent sequences. Thus, the term "enzyme variant" or "sequence variant" or "protein variant" are used in reference to parent enzymes that are the origin for the respective variant enzymes. Therefore, parent enzymes include wild type enzymes and variants of wild-type enzymes which are used for development of further variants. Variant enzymes differ from parent enzymes in their amino acid sequence to a certain extent.

"Amino acid substitutions" are described by providing the original amino acid followed by the number of the position within the amino acid sequence, followed by the substituted amino acid. For example, the substitution of histidine at position 120 with alanine is designated as "His120Ala" or "H120A". Substitutions can also be described by merely naming the resulting amino acid in the variant without specifying the amino acid of the parent sequence at this position, e.g., by using "X120A" or "120A" or "Xaa120Ala" or "120Ala".

Variants comprising multiple substitutions are separated by "+", e.g., "Arg170Tyr+Gly195Glu", "R170Y+G195E" or "X170Y+X195E" representing a substitution of arginine and glycine at positions 170 and 195 with tyrosine and glutamic acid, respectively. Alternatively, multiple substitutions may be separated by space or a comma, e.g., "R170Y G195E" or "R170Y, G195E" respectively. Where different alternative substitutions can be introduced at a position, the different substitutions are separated by a comma, e.g., "Arg170Tyr,Glu" and "R170T,E", respectively, represents a substitution of arginine at position 170 with tyrosine or glutamic acid. Alternative substitutions at a particular position can also be indicated as "X120A,G,H", "120A,G,H", "X120A/G/H", or "120A/G/H". Alternatively, different substitutions may be indicated in brackets, e.g., "Arg170[Tyr,Gly]" or "Arg170{Tyr,Gly}" or in short "R170 [Y,G]" or "R170 {Y,G}". The numbering of the amino acid residues of the proteases described herein is as commonly used for proteases in the field (cf. P.N. Bryan, Biochimica et Biophysica Acta 1543 (2000), 203-222, cf. p. 204, left col., 3rd para.) according to the numbering of the BPN' subtilisin protease from Bacillus amyloliquefaciens (i.e., according to "BPN' numbering", i.e., according to the numbering of SEQ ID NO: 2 of the present invention).

Variant polynucleotide and variant polypeptide sequences may be defined by their sequence identity when compared to a parent sequence. Sequence identity usually is provided as "% sequence identity" or "% identity". For calculation of sequence identities, in a first step a sequence alignment is produced. According to this invention, a pairwise global alignment is produced, meaning that two sequences are aligned over their complete length, which is usually produced by using a mathematical approach, called alignment algorithm.

According to the invention, the alignment is generated by using the algorithm of Needleman and Wunsch (J. Mol. Biol. (1970) 48, p. 443-453). Preferably, the program "NEEDLE" (The European Molecular Biology Open Software Suite (EMBOSS)) is used for the purposes of the current invention, with using the programs default parameter (polynucleotides: gap open=10.0, gap extend=0.5 and matrix=EDNAFULL; polypeptides: gap open=10.0, gap extend=0.5 and matrix=EBLOSUM62). After aligning two sequences, in a second step, an identity value is determined from the alignment produced. For this purpose, the %-identity is calculated by dividing the number of identical residues by the length of the alignment region which is showing the respective sequence of the present invention over its complete length multiplied with 100: %-identity = (identical residues / length of the alignment region which is showing the respective sequence of the present invention over its complete length) *100.

A special aspect concerning amino acid substitutions are conservative mutations which often appear to have a minimal effect on protein folding resulting in substantially maintained enzyme properties of the respective enzyme variant compared to the enzyme properties of the parent enzyme. Conservative mutations are those where one amino acid is exchanged with a similar amino acid. Such an exchange most probably does not change enzyme properties. Herein the following conservative exchanges are considered:
Amino acid A is similar to amino acids S
Amino acid D is similar to amino acids E; N
Amino acid E is similar to amino acids D; K; Q
Amino acid F is similar to amino acids W; Y
Amino acid H is similar to amino acids N; Y
Amino acid I is similar to amino acids L; M; V
Amino acid K is similar to amino acids E; Q; R
Amino acid L is similar to amino acids I; M; V
Amino acid M is similar to amino acids I; L; V
Amino acid N is similar to amino acids D; H; S
Amino acid Q is similar to amino acids E; K; R
Amino acid R is similar to amino acids K; Q
Amino acid S is similar to amino acids A; N; T
Amino acid T is similar to amino acids S
Amino acid V is similar to amino acids I; L; M
Amino acid W is similar to amino acids F; Y
Amino acid Y is similar to amino acids F; H; W

Conservative amino acid substitutions may occur over the full length of the sequence of a polypeptide sequence of a functional protein such as an enzyme. Preferably, such mutations are not pertaining the functional domains of an enzyme, more preferably conservative mutations are not pertaining the catalytic centers of an enzyme.

A "fragment" or "subsequence" as used herein refers to a portion of an amino acid sequence. Polypeptides comprising a deletion of one or more amino acids at the N terminus and/or the C terminus of the polypeptide and essentially retain protease activity are herein designated as "functional fragments". Preferably, the functional fragment has at least 50%, at least 55%, at least 60%, at least 65%, at least 70%, at least 75%, at least 76%, at least 77%, at least 78%, at least 79%, at least 80% identical, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 98.5 %, at least 99%, or at least 99.5% of the length of the original full length amino acid sequence. The functional fragment comprises consecutive amino acids compared to the original full length amino acid sequence.

The compositions according to the invention may comprise one or more compounds, which may form water-soluble complexes with ions, in particular calcium and magnesium ions. Such compound may be called "builder" or "building agent" or "chelating agent" or "complexing agent" herein.

A "sequestering builder" as used herein is different from a precipitating builder in that no significant amount of precipitate is formed when the builder is used in an amount sufficient to combine with all of the calcium ions in an aqueous solution with 7 °dH hardness (German hardness) initially at neutral pH. A "strong builder" is classified as high efficiency chelators that can bind the divalent cations such as Ca2+ strongly with a logarithmic stability constant (Log KCa) of the cation/chelator complex of above 4, particular above 5, above 6 or above 7. The stability constants are determined at an ionic strength of 0.1 M and at a temperature of 25°C. A"strong sequestering build-er" combines both of the above-mentioned properties.

An "antimicrobial agent" is a chemical compound that kills microorganisms or inhibits their growth or reproduction. Microorganisms can be bacteria, yeasts or molds. A "preservative" is an antimicrobial agent which may be added to aqueous products and compositions to maintain the original performance, characteristics and integrity of the products and compositions by killing contaminating microorganisms or inhibiting their growth.

A "hair-care composition" as used herein refers to a specific formulation or combination of substances intended for use in the treatment and maintenance of hair health and appearance. The hair-care composition can be in the form of a shampoo, serum, spray, gel, or any other product designed for application to the hair and skin. Its primary purpose is to cleanse, nourish, protect, enhance, or style the hair, addressing various concerns such as dryness, damage, frizz, volume, shine, or color preservation.

A "rinse-off composition", as a specific type of hair-care composition, refers to a hair-care composition that is designed to be applied to the hair and skin and then rinsed off with water after a specified period.

A "shampoo composition" (also called herein "hair shampoo" or "shampoo"), as a specific type of hair-care composition, is a cleansing product specifically formulated for the purpose of removing dirt, excess oil, product buildup, and impurities from the hair and skin. It typically comes in liquid or gel form, and is applied to wet hair, lathered, and then rinsed out. A shampoo composition can also be in solid form and used as a soap bar that is partially solubilized when rubbed into the wet hair. Typically, the application of the shampoo composition to the wet hair is associated with foam formation due to the surfactants contained in the composition.

A "conditioning agent" (also called herein "conditioner") is a typical compound contained in hair-care compositions that works by depositing a film or coating onto the hair strands, which at least temporarily stays on the hair surface after rinsing-off the composition ("leave-in"), which thereby helps to smooth the hair cuticles, reduce frizz, and detangle the hair and thus reduce the force needed for hair combing. A "synthetic conditioning agent" is a conditioning agent that is not natural, i.e., a compound made by humans. Examples of such synthetic conditioning agents are polymeric siloxanes or quaternary ammonium compounds.

"Non-synthetic conditioning agents" are naturally derived compounds or derivatives thereof with hair-conditioning effect. Examples of such non-synthetic conditioning agents are natural oils, e.g., coconut oils, and natural waxes, e.g., candelilla wax, and their hydrogenation and/or esterification products, e.g., hardened rape seed oil or ethylene glycol distearate.

A composition "essentially devoid" of a compound shall mean herein that the respective compound is not added to the composition on purpose, meaning that at most non-effective amounts are present, most preferably 0% of the compound are contained in the composition.

Unless otherwise stated herein, an amount of a compound given in percent (%) is referring to the mass fraction in weight percent (wt.-%) of the respective compound.

"Solid" as used herein describes an aggregate state that is neither gaseous nor liquid.

"Liquid" is defined herein as having a viscosity of 500-30000 mPa*sec determined using a Brookfield viscometer at a temperature of 20°C, with spindle 4 rotating at a speed of 10 revolutions per minute (rpm).

"Polymeric siloxanes" (herein also called "silicones") are polymers that consist of repeating units of silicium and oxygen atoms, with various organic groups, e.g., methyl groups, attached to the silicium atoms.

"Quaternary ammonium compounds" (also referred to as QAC or quats) are organic ammonium compounds in which all four valences of the nitrogen atom are bound to organic groups. Therefore, quaternary ammonium compounds are salts, which means they are ionic compounds consisting of a cation and an anion. There are two types of quaternary ammonium compounds: the amine type NR4+X-, in which all four R groups are organic residues, and the imine type R=NR2+X-, in which all three R groups are organic residues, wherein X- is the corresponding anion. N-alkylated heteroaromatics also belong to the quaternary ammonium compounds. Quaternary ammonium compounds can be monomeric or polymeric. Polymeric quaternary ammonium compounds (also called polyquaternium polymers or poly-quats) are synthetic polymers that contain quaternary ammonium groups with organic side chains linked via alkyl groups.

A "biodegradable" compound (such as a surfactant or complexing agent) is a compound that is readily biodegradable in an aerobic aqueous medium according to the OECD guideline 301. "Readily biodegradable" is defined according to the Detergents Regulation as the ability of a product to biodegrade more than 60% within 28 days according to OECD 301A-F/ASTM. "Biobased" refers to a product or material that is derived from renewable biological resources such as plants, animals, and microorganisms, rather than from non-renewable fossil-based resources.

### Compositions

The composition of the present invention comprises one or more protease, and one or more surfactant.

Preferably, the composition of the present invention further comprises one or more salts.

Preferably, the composition is essentially devoid of alkyl dimethyl polysiloxanes. Preferably, the composition is essentially devoid of dimethyl polysiloxanes, preferably the composition is essentially devoid of polymeric siloxanes, preferably essentially devoid of polymeric siloxanes and quaternary ammonium compounds, preferably polyquaternium polymers. Preferably, the composition is essentially devoid of a synthetic conditioning agent, more preferably devoid of any conditioning agent.

Preferably, the pH of the composition is pH 4.0-8.5.

The composition of the present invention may further comprise one or more component selected from the group consisting of solubilizer, thickener, biopolymer, UV filters, chelating agent, preservative, pH adjusters, enzyme stabilizing system, fragrances, botanical extracts, and humectants.

Preferably one or more of the components of the composition, preferably the one or more surfactants, are bio-degradable and/or bio-based, preferably bio-degradable and bio-based. Most preferably, all of the components of the composition are bio-degradable and/or bio-based, preferably bio-degradable and bio-based.

The composition of the present invention is preferably a hair-care composition, preferably a rinse off composition, preferably a shampoo composition.

The composition of the present invention can either be solid or liquid. Solid compositions may be in the form of powder, bar, or sheet.

As outlined above, the present invention is based on the discovery that proteases can be used to smoothen the hair surface and therefore to reduce the need to add leave-in conditioning agents, in particular non-biodegradable polymeric siloxanes. Thus, the hair-care composition of the present invention provides hair-conditioning effects without the need for leave-in-conditioners. Thus, in one embodiment, the present invention is directed to a rinse-off hair-conditioning composition or rinse-off conditioning composition with properties as described herein.

### Protease

The protease of the composition of the present invention can be any protease. Proteases according to the invention have "proteolytic activity" (also referred to as "protease activity"). This property is related to hydrolytic activity of a protease (i.e., proteolysis, which means hydrolysis of peptide bonds linking amino acids together in a polypeptide chain) on protein containing substrates, e.g., casein, haemoglobin, and BSA. Quantitatively, proteolytic activity is related to the rate of degradation of protein by a protease or proteolytic enzyme in a defined course of time. The methods for analyzing proteolytic activity are well-known in the literature (see e.g. Gupta et al. (2002), Appl. Microbiol. Biotechnol. 60: 381-395). For instance, proteolytic activity can be determined by using Succinyl-Ala-Ala-Pro-Phe-p-nitroanilide (Suc-AAPF-pNA, short AAPF; see e.g. DelMar et al. (1979), Analytical Biochem 99, 316-320) as substrate. pNA is cleaved from the substrate molecule by proteolytic cleavage, resulting in release of yellow color of free pNA which can be quantified by measuring OD405. To determine changes in proteolytic activity over time, the "initial enzymatic activity" of a protease is measured under defined conditions at time zero and at a certain point in time later. By dividing the latter activity with the activity at time point zero the residual activity can be calculated (x%). By comparison of the 100%-value with the x%-value, a potential loss of proteolytic activity can be determined in its extent.

Preferably, the amount of the protease in the composition is 0.001 to 1.5 wt.-%, preferably 0.001 to 1 wt.-%. Preferably, the protease is capable of degrading keratin. Preferably, the protease comprises as a catalytic triad the amino acids aspartate, histidine, and serine. Preferably, the protease is a subtilisin protease.

Preferably, the protease of the present invention is a variant protease of the parent protease shown in SEQ ID NO: 1. Preferably, the variant protease comprises an amino acid sequence which is at least 60%, preferably at least 80%, identical to SEQ ID NO: 1 and wherein the amino acid sequence of the protease comprises compared to SEQ ID NO: 1 at least one, preferably at least two, additional negative charges in the loop region of residues 98 to 104 according to the numbering of SEQ ID NO: 2 (BPN' numbering, i.e., wherein the positions are numbered by their correspondence to the amino acid sequence of subtilisin BPN' of B. amyloliquefaciens, herein shown as SEQ ID NO: 2). Preferably, the protease has at least 60%, preferably at least 80%, sequence identity to SEQ ID NO: 1 as described herein and comprises compared to SEQ ID NO: 1 at least two, three, or four additional negative charges, more preferably three additional negative charges, most preferably two additional negative charges in the loop region of residues 98 to 104 according to the numbering of SEQ ID NO: 2 compared to the region of SEQ ID NO: 1 corresponding to residues 98 to 104 according to the numbering of SEQ ID NO: 2.

Preferably, the at least one, preferably at least two, additional negative charges in the loop region of residues 98 to 104 according to the numbering of SEQ ID NO: 2 are obtained by one or more amino acid alterations selected from the group consisting of substitutions, deletions and insertions, preferably by substitutions.

Preferably, in the protease the at least one, preferably at least two, additional negative charges compared to SEQ ID NO: 1 in the loop region of residues 98 to 104 are caused by one or more amino acid substitutions at amino acid position selected from the group consisting of 98, 99, 100, 101, 102, 103, and 104, preferably at position 101, according to the numbering of SEQ ID NO: 2.

Preferably, the at least one, preferably at least two, additional negative charges in the loop region of residues 98 to 104 according to the numbering of SEQ ID NO: 2 are obtained by one or more amino acid alterations selected from the group consisting of D99E, R101D and R101E.

In a preferred embodiment, the protease comprises an amino acid sequence which comprises compared to SEQ ID NO: 1 the amino acid substitution R101E or R101D, preferably R101E, according to the numbering of SEQ ID NO: 2. In another preferred embodiment, the at least one, preferably at least two, additional negative charges compared to SEQ ID NO: 1 in the loop region of residues 98 to 104 are not caused by the amino acid substitution R101E or R101D.

In a preferred embodiment, the loop sequence 98-104 that has compared to SEQ ID NO: 1 two additional negative charges comprises a sequence selected from the group consisting of ADGEGAI, ADGDGAI, ADGDGSV, ADGEGSV, AADGEGSV, and ASEGEGSV with longer sequences having an insertion in the loop sequence.

In one embodiment, the protease has at least 60%, at least 65%, at least 70%, at least 75%, preferably, at least 80%, at least 85%, at least 90%, at least 95%, at least 96%, at least 97%, at least 98%, or at least 99%, but less than 100% sequence identity to SEQ ID NO: 1 and comprises compared to SEQ ID NO: 1 at least one, preferably at least two, additional negative charges in the loop region of residues 98 to 104 according to the numbering of SEQ ID NO: 2. Preferably, the protease has at least 60%, at least 65%, at least 70%, at least 75%, preferably, at least 80%, at least 85%, at least 90%, at least 95%, at least 96%, at least 97%, at least 98%, or at least 99%, but less than 100% sequence identity to SEQ ID NO: 1 and comprises compared to SEQ ID NO: 1 the amino acid substitution R101D or R101E, preferably R101E, according to the numbering of SEQ ID NO: 2. Preferably, the protease has at least 85%, at least 90%, at least 95%, at least 96%, at least 97%, at least 98%, or at least 99%, but less than 100% sequence identity to SEQ ID NO: 1 and comprises compared to SEQ ID NO: 1 the amino acid substitution R101E according to the numbering of SEQ ID NO: 2. Preferably, the protease has 100% sequence identity to SEQ ID NO: 1 and comprises compared to SEQ ID NO: 1 the amino acid substitution R101D or R101E, preferably R101E, according to the numbering of SEQ ID NO: 2, i.e., the protease comprises compared to SEQ ID NO: 1 only one amino acid exchange.

Further preferred proteases can comprise an amino acid sequence having with increasing preference at least 80%, at least 85%, at least 90%, at least 91%, at least 92%, at least 93%, at least 94%, at least 95%, at least 96%, at least 97%, at least 98%, or at least 99%, but below 100% sequence identity with SEQ ID NO:22 as described in EP 1921147 (SEQ ID NO: 1 of the present invention) and comprising amino acid substitutions in one or more of the following positions 3, 4, 9, 15, 27, 33, 36, 57, 68, 77, 87, 95, 96, 97, 98, 99, 100, 101, 102, 103, 104, 106, 118, 120, 123, 128, 129, 130, 131, 154, 160, 167, 170, 194, 199, 205, 206, 217, 218, 222, 224, 232, 235, 236, 245, 252 and 274 (according to the BPN' numbering, i.e., according to the numbering of SEQ ID NO: 2 of the present invention), which has proteolytic activity. In one embodiment, such a protease is not mutated at positions Asp32, His64 and Ser221 (according to the BPN' numbering, i.e., according to the numbering of SEQ ID NO: 2 of the present invention).

Preferably, the protease comprises an amino acid sequence having with increasing preference at least 80%, at least 85%, at least 90%, at least 91%, at least 92%, at least 93%, at least 94%, at least 95%, at least 96%, at least 97%, at least 98%, or at least 99%, but below 100% sequence identity with SEQ ID NO:22 as described in EP 1921147 (SEQ ID NO: 1 of the present invention) and is further characterized by having amino acid glutamic acid (E), or aspartic acid (D), or asparagine (N), or glutamine (Q), or alanine (A), or glycine (G), or serine (S), preferably glutamic acid (E), at position 101 (according to the BPN' numbering, i.e., according to the numbering of SEQ ID NO: 2 of the present invention) and has proteolytic activity. Mostly preferred is a protease which has at least 80%, but below 100% sequence identity with SEQ ID NO:22 as described in EP 1921147 (SEQ ID NO: 1 of the present invention) and is characterized by having amino acid glutamic acid (E) at position 101 (according to the BPN' numbering, i.e., according to the numbering of SEQ ID NO: 2 of the present invention) and has proteolytic activity. The protease may comprise an amino acid substitution at position 101, such as R101E, alone or in combination with one or more substitutions at positions 3, 4, 9, 15, 27, 33, 36, 57, 68, 77, 87, 95, 96, 97, 98, 99, 100, 102, 103, 104, 106, 118, 120, 123, 128, 129, 130, 131, 154, 160, 167, 170, 194, 199, 205, 206, 217, 218, 222, 224, 232, 235, 236, 245, 252 and/or 274 (according to the BPN' numbering, i.e., according to the numbering of SEQ ID NO: 2 of the present invention) and has proteolytic activity. In one embodiment, said protease comprises one or more further substitutions: (a) threonine at position 3 (3T), (b) isoleucine at position 4 (4I), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or 217G), (i) combinations of two or more amino acids according to (a) to (h), according to the BPN' numbering, i.e., according to the numbering of SEQ ID NO: 2 of the present invention. A suitable protease may be at least 80% identical to SEQ ID NO:22 as described in EP 1921147 (SEQ ID NO: 1 of the present invention) and is characterized by comprising one amino acid (according to (a)-(h)) or combinations according to (i) together with the amino acid 101E, 101D, 101N, 101Q, 101A, 101G, or 101S (according to the BPN' numbering, i.e., according to the numbering of SEQ ID NO: 2 of the present invention) and has proteolytic activity. In one embodiment, the protease is at least 80% identical to SEQ ID NO:22 as described in EP 1921147 (SEQ ID NO: 1 of the present invention) and is characterized by comprising the mutation (according to the BPN' numbering, i.e., according to the numbering of SEQ ID NO: 2 of the present invention) R101E, or S3T +V4I +V205I, or S3T +V4I + R101E + V205I or S3T + V4I + V199M + V205I + L217D, and has proteolytic activity. In another embodiment, the protease comprises an amino acid sequence having at least 80% identity to SEQ ID NO:22 as described in EP 1921147 (SEQ ID NO: 1 of the present invention) and being further characterized by comprising S3T + V4I + S9R + A15T + V68A + D99S + R101S +A103S + I104V + N218D (according to the BPN' numbering, i.e., according to the numbering of SEQ ID NO: 2 of the present invention) and has proteolytic activity. In another embodiment, the protease may have an amino acid sequence being at least 80% identical to SEQ ID NO:22 as described in EP 1921147 (SEQ ID NO: 1 of the present invention) and being further characterized by comprising R101E, and one or more substitutions selected from the group consisting of S156D, L262E, Q137H, S3T, R45E,D,Q, P55N, T58W,Y,L, Q59D,M,N,T, G61D,R, S87E, G97S, A98D,E,R, S106A,W, N117E, H120V,D,K,N, S125M, P129D, E136Q, S144W, S161T, S163A,G, Y171L, A172S, N185Q, V199M, Y209W, M222Q, N238H, V244T, N261T,D and L262N,Q,D (according to the BPN' numbering, i.e., according to the numbering of SEQ ID NO: 2 of the present invention), and has proteolytic activity. In this embodiment, preferably, the protease comprises an amino acid sequence which is at least 80%, at least 85%, at least 90%, at least 95%, at least 96%, at least 97%, at least 98%, or at least 99%, but less than 100% identical to SEQ ID NO:22 as described in EP 1921147 (SEQ ID NO: 1 of the present invention) and the protease comprises compared to SEQ ID NO:22 as described in EP 1921147 (SEQ ID NO: 1 of the present invention) the amino acid substitutions R101E and S156D and/or L262E, and optionally at least one further mutation selected from I104T, H120D, Q137H, S141H, R145H and S163G according to the BPN' numbering, i.e., according to the numbering of SEQ ID NO: 2 of the present invention.

In another embodiment, the protease used in combination with the polypeptide having protease activity has at least 60%, preferably at least 80%, at least 85%, at least 90%, at least 95%, at least 96%, at least 97%, at least 98%, or at least 99%, but less than 100% sequence identity to SEQ ID NO:22 as described in EP 1921147 (SEQ ID NO: 1 of the present invention) and comprises compared to SEQ ID NO:22 as described in EP 1921147 (SEQ ID NO: 1 of the present invention) the amino acid substitution R101D or R101E, preferably R101E, and one or more of the amino acid substitutions selected from the group consisting of S3T, V4I, and V205I, preferably all of the amino acid substitutions S3T, V4I, and V205I, according to the BPN' numbering, i.e., according to the numbering of SEQ ID NO: 2 of the present invention, and one or more substitutions at positions according to the BPN' numbering, i.e., according to the numbering of SEQ ID NO: 2 of the present invention, selected from the group consisting of 76, 138, 145, 156, 166, 167, 169, 177, 187, 189, 191, 206, 209, 215, 218, and 262, preferably selected from the group consisting of N76D/E/Q, A138Q, R145L/W/Y, S156D/Q, S166G, Y167T, A169G, V177 L, A187 D, F189R, Q191R, Q206A/L/S/T, Y209K/V/W, A215K/W, N218S/T, and L262D/E/Q.

In this embodiment, preferably, the protease used in combination with the polypeptide having protease activity has at least 80%, at least 85%, at least 90%, at least 95%, at least 96%, at least 97%, at least 98%, or at least 99%, but less than 100% sequence identity to SEQ ID NO:22 as described in EP 1921147 (SEQ ID NO: 1 of the present invention) and comprises compared to SEQ ID NO:22 as described in EP 1921147 (SEQ ID NO: 1 of the present invention) the amino acid substitution R101D or R101E, preferably R101E, and one or more of the amino acid substitutions selected from the group consisting of S3T, V4I, and V205I, preferably all of the amino acid substitutions S3T, V4I, and V205I, according to the BPN' numbering, i.e., according to the numbering of SEQ ID NO: 2 of the present invention, and one or more substitutions at positions according to the BPN' numbering, i.e., according to the numbering of SEQ ID NO: 2 of the present invention, selected from the group consisting of 156, 166, 187, 189, 191, 206, 209, 215 and 262, preferably selected from the group consisting of S156D, S166G, A187 D, F189R, Q191R, Q206L, Y209W, A215K und L262E. In this embodiment, particularly preferred, the protease used in combination with the polypeptide having protease activity has at least 80%, at least 85%, at least 90%, at least 95%, at least 96%, at least 97%, at least 98%, or at least 99%, but less than 100% sequence identity to SEQ ID NO:22 as described in EP 1921147 (SEQ ID NO: 1 of the present invention) and comprises compared to SEQ ID NO:22 as described in EP 1921147 (SEQ ID NO: 1 of the present invention) according to the BPN' numbering, i.e., according to the numbering of SEQ ID NO: 2 of the present invention, the amino acid substitutions
(i) S3T-V4I-R101E-V199I-Q206L-Y209W;
(ii) S3T-V4I-R101E-V199I-N218S;
(iii) S3T-V4I-R101E-V199I-N76D;
(iv) S3T-V4I-R101E-V199I-S156D-L262E;
(v) S3T-V4I-R101E-V199I-Q206L-Y209W-S156D-L262E;
(vi) S3T-V41-R101E-V199I-N76D-Q206L-Y209W;
(vii) S3T-V4I-R101E-V199I-N76D-S156D-Q206L-Y209W-L262E;
(viii) S3T-V4I-R101E-V199I-N76D-N218S;
(ix) S3T-V4I-R101E-V199I-N760-S1560-Y209W-L262E;
(x) S3T-V4I-R101E-V199I-N76D-Y209W;
(xi) S3T-V4I-R101E-V199I-N760-S156D-Q206L-L262E;
(xii) S3T-V4I-R101E-V199I-N76D-Q206L;
(xiii) S3T-V4I-R101E-V199I-S156D-Q206L-Y209W;
(xiv) S3T-V41-R102E-V199I-Q206L-Y209W-L262E;
(xv) S3T-V4I-R101E-V199I-A138Q-R145W-Y167 T-Q206L;
(xvi) S3T-V4I-R101E-V199I-N76D-R145Y-A215W-N218S-L262E;
(xvii)S3T-V4I-R101E-V199I-A138Q-S156D-V177 L-Q206L;
(xviii) S3T-V4I-R101E-V199I-Q206L-Y209W-A215K-S156D-L262E;
(xix) S3T-V4I-R101E-V199I-S156D-S166G-Q191R-Q206L-Y209W-L262E; or
(xx) S3T-V4I-R101E-V199I-S156D-A187 D-F189R-Q206L-Y209W-L262E.

Most preferably, the protease has at least 80%, at least 85%, at least 90%, at least 95%, at least 96%, at least 97%, at least 98%, or at least 99%, but less than 100% sequence identity to SEQ ID NO: 1 and comprises compared to SEQ ID NO: 1 the amino acid substitution R101E according to the numbering of SEQ ID NO: 2, preferably, the protease has 100% sequence identity to SEQ ID NO: 1 and comprises compared to SEQ ID NO: 1 the amino acid substitution R101E according to the numbering of SEQ ID NO: 2, i.e., the protease comprises compared to SEQ ID NO: 1 only one amino acid exchange.

Commercially available protease enzymes include but are not limited to those sold under the trade names Progress^{®} Uno, Alcalase^{®}, Blaze^{®}, Duralase^{™}, Durazym^{™}, Relase^{®}, Relase^{®} Ultra, Savinase^{®}, Savinase^{®} Ultra, Primase^{®}, Polarzyme^{®}, Kannase^{®}, Liquanase^{®}, Liquanase^{®} Ultra, Ovozyme^{®}, Coronase^{®}, Coronase^{®} Ultra, Neutrase^{®}, Everlase^{®} and Esperase^{®} (Novozymes A/S) and those sold under the tradename Maxatase^{®}, Maxacal^{®}, Maxapem^{®}, Purafect^{®}, Purafect^{®} Prime, Purafect MA^{®}, Purafect Ox^{®}, Purafect OxP^{®}, Puramax^{®}, Properase^{®}, FN2^{®}, FN3^{®}, FN4^{®}, Excellase^{®}, Eraser^{®}, Ultimase^{®}, Opticlean^{®}, Effectenz^{®}, Preferenz^{®} (P100, P200, P300 and P400 from IFF) and Optimase^{®} (Danisco/DuPont), Axapem^{™} (Gist-Brocases N.V.), KAP (Bacillus alkalophilus subtilisin) from Kao Corporation and Lavergy Pro (BASF).

### Surfactants

In one embodiment, the compositions according to the invention comprise one or more surfactant(s). According to its ionic charge, a surfactant is called non-ionic, anionic, cationic, or amphoteric.

The composition of the present invention may comprise one or more surfactants, which may be anionic and/or cationic and/or non-ionic and/or amphoteric, or a mixture thereof. In a preferred embodiment, the compositions of the invention comprise at least one surfactant. In a particular embodiment, the composition of the present invention includes a mixture of one or more amphoteric, one or more non-ionic surfactants, and one or more ionic surfactants. The total amount of surfactants in the composition can range from 1 to 95 wt.-%, depending on the type of composition. The surfactant(s) is/are typically present at a level of from about 1 to 60 wt.-%, 1 to 40 wt.-%, preferably 3 to 30 wt.-%, 5 to 30 wt.-%, or 8 to 30 wt.-%, in particular in liquid compositions.

For solid compositions, the surfactant(s) is/are typically present at a level of from about 35 to 95 wt.-%, preferably, 40 to 80 wt.-%, 45 to 80 wt.-% or 50 to 80 wt.-%.

The surfactant(s) is/are chosen based on the desired hair-care application, and includes any conventional surfactant(s) known in the art. Non-limiting examples of surfactants are disclosed in McCutcheon's 2016 Detergents and Emulsifiers, and McCutcheon's 2016 Functional Materials, both North American and International Edition, MC Publishing Co, 2016 edition. Further useful examples are disclosed in earlier editions of the same publications which are known to those skilled in the art. Surfactants particularly suitable for the composition of the present invention are outlined below.

### Ionic surfactants

The composition of the preset invention preferably comprises one or more ionic surfactants, preferably an anionic surfactant and/or a cationic surfactant.

The total amount of ionic surfactants in the composition can range from 0 to 95 wt.-%, depending on the type of composition. In liquid compositions, the total amount of ionic surfactants is preferably from 0 to 30 wt.-%, preferably 1 to 30 wt.-%, 5-30 wt.-%, or 8 to 30 wt.-%.

In solid compositions, the total amount of ionic surfactants is preferably from 15 to 95 wt.-%, preferably, 35 to 95 wt.-%, 35 to 80 wt.-% or 45 to 80 wt.-%.

Preferably, the composition of the present invention preferably comprises one or more anionic surfactants. Non-limiting examples of anionic surfactants include sulfates and sulfonates, in particular linear alkyl benzene sulfonates (LAS), isomers of LAS, alpha-olefin sulfonates (AOS), olefin sulfonates, alkene sulfonates, alkane-2,3-diylbis(sulfates), hydroxy alkane sulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkane sulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenyl succinic acid, dodecenyl/tetradecenyl succinic acid (DTSA), fatty acid derivatives of amino acids, diesters and monoesters of sulfo succinic acid or soap, and combinations thereof.

Preferably, the one or more anionic surfactants are selected from the group consisting of secondary alkansulfonates, alkylsulfates, alkylethersulfates, olefinsulfonates, alkylamidethersulfates, acylisothienates, acylglutamates, alkylethercarboxylates, methyltauride and taurades, sarkosides, sulfosuccinates, protein fatty acid condensates, salt of alkyl hydrolyzed collagen, potassium-cocoyl hydrolyzed collagen, TEA-cocoyl hydrolyzed collagen, and combinations thereof.

Preferably, the composition comprises one or more surfactants selected from the group consisting of monovalent and bivalent alkylsulfates, e.g. sodiumalkylsulfate, sodium-alkyl-ether sulfates, sulfosuccinates, alkyl isothienate, soap, and combinations thereof. Preferably, the composition comprises an alkylethersulfate, preferably sodium laurylethersulfate.

Preferably, the one or more anionic surfactants are selected from the group consisting of monovalent and bivalent alkylsulfates, e.g. sodiumalkylsulfate, sodium-alkyl-ether sulfates, sulfosuccinates, alkyl isothienate, soap, and combinations thereof. Preferably, the anionic surfactant is an alkylethersulfate, preferably sodium laurylethersulfate. Non-limiting examples of cationic surfactants include alkyl dimethyl ethanolamine quat (ADMEAQ), cetyl trimethyl am-monium bromide (CTAB), dimethyl distearyl ammonium chloride (DSDMAC), alkyl benzyl dimethyl ammonium, alkyl quaternary ammonium compounds, alkoxylated quaternary ammonium (AQA) compounds, and combinations thereof. Preferably, the composition comprises a surfactant selected from the group consisting of alkyl dimethyl ethanolamine quat (ADMEAQ), cetyl trimethyl ammonium bromide (CTAB), dimethyl distearyl ammonium chloride (DSDMAC), alkyl benzyl dimethyl ammonium, alkyl quaternary ammonium compounds, alkoxylated quaternary ammonium (AQA) compounds, and combinations thereof.

### Amphoteric surfactant

The composition of the present invention preferably comprises one or more amphoteric surfactants.

The total amount of ionic surfactants in the composition can range from 0 to 95 wt.-%, depending on the type of composition. In liquid compositions, the total amount of ionic surfactants is preferably from 0 to 30 wt.-%, preferably 1 to 30 wt.-%, 5-30 wt.-%, or 8 to 30 wt.-%.

In solid compositions, the total amount of ionic surfactants is preferably from 10 to 95 wt.-%, preferably, 35 to 95 wt.-%, 35 to 80 wt.-% or 45 to 80 wt.-%.

Preferably, the one or more amphoteric surfactant are selected from the group consisting of alkylbetaine, alkylamidobetaine, sulfobetaines, alkyl-amphoacetates, alkyl-amphodiacetates, imidazolines, coco-amphopropionates, and combinations thereof.

Preferably, the composition comprises a surfactant selected from the group consisting of betaine, alkyl dimethyl betaine, sulfo betaine, and combinations thereof, preferably the composition comprises cocoamidoproylbetaine. Preferably, the amphoteric surfactant is selected from the group consisting of betaine, alkyl dimethyl betaine, sulfo betaine, and combinations thereof. Preferably the amphoteric surfactant is cocoamidoproylbetaine.

### Non-ionic surfactants

The composition of the preset invention preferably comprises one or more non-ionic surfactants.

The total amount of ionic surfactants in the composition can range from 0 to 95 wt.-%, depending on the type of composition. In liquid compositions, the total amount of ionic surfactants is preferably from 0 to 30 wt.-%, preferably 1 to 30 wt.-%, 5 to 30 wt.-%, or 8 to 30 wt.-%.

In solid compositions, the total amount of ionic surfactants is preferably from 10 to 95 wt.-%, preferably, 35 to 95 wt.-%, 35 to 80 wt.-% or 45 to 80 wt.-%.

Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, , alkyl polyglycosides (APG), alkoxylated amines, fatty acid monoethanolamides (FAM), fatty acid diethanol amides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN and Brji (polyalkylenglycolether), and combinations thereof.

Preferably, the composition comprises one or more surfactants are selected from the group consisting of ethoxilated ricinol-derivatives, polysorbates, gylcerol fatty acid esters, ethoxylated alkylphenolpolyglycolethers, alkyl polyglucosides (APGs), alkyl-aminoxides, and combinations thereof.

Preferably, the one or more non-ionic surfactants are selected from the group consisting of ethoxilated ricinol-derivatives, polysorbates, gylcerol fatty acid esters, ethoxylated alkylphenolpolyglycolethers, alkyl polyglucosides (APGs), alkyl-aminoxides, and combinations thereof.

Preferably, the composition comprises a surfactant selected from the group consisting of an alkylpolyglucoside (APG), sorbitan-esters, sorbitan-derivative, and combinations thereof.

Preferably, the non-ionic surfactant is selected from the group consisting of an alkylpolyglucoside (APG), a sorbitan-esters, sorbitan-derivative, and combinations thereof.

Preferably, one or more, preferably all, surfactants in the composition are biobased and/or biodegradable. More preferably, one or more, preferably all, surfactants in the composition are biobased and biodegradable. A biodegradable surfactant is readily biodegradable in an aerobic aqueous medium according to the OECD guideline 301. Biodegradable surfactants include but are not limited to, sodium alkyl ether sulfates, preferably sodium laureth sulfate, preferably sodium laureth sulfate + 2 EO (Texapon N70), alkyl polyglucosides, preferably alkyl polyglucoside C10-C16 (Glucopon 600), and alcohol ethoxylates, preferably C12-C18 fatty alcohol + 7 EO (Dehydol LT 7).

### Salt

The composition of the present invention comprises one or more salts. Non-limiting examples of salts include NaCl, KCI, MgCl2, CaCl2, Na2SO4, MgSO4, and combinations thereof. Preferably, the composition comprises a salt selected from the group consisting of NaCl, KCI, MgCl2, CaCl2, Na2SO4, MgSO4, and combinations thereof. Preferably, the salt is selected from NaCl, KCI, MgCl2, CaCl2, Na2SO4, MgSO4, and combinations thereof. Preferably, the amount of salt in the composition is 0.1 to 5 wt.-%, more preferably 0.5 to 4 wt.-%.

### Other components

The composition of the present invention may further comprise one or more component selected from the group consisting of solubilizer, thickener, biopolymer, UV-filters, chelating agent, preservative, pH adjusters, enzyme stabilizing system, fragrances, botanical extracts, and humectants.

In one embodiment, the composition of the present invention comprises one or more solubilizer. The solubilizer is added to create a stable and/or clear formulation.

Solubilizers may be added to the composition in an amount of 0.1 to 5 wt.-%.

Common solubilizers include Tween 80, ethylhexylsulfonate, PEG40-hydrogenated castor-oil, and combinations thereof. Preferably, the composition comprises a compound selected from the group consisting of Tween 80, ethylhexylsulfonate, PEG40-hydrogenated castor-oil, and combinations thereof. Preferably, the composition comprises PEG40-hydrogenated castor-oil. Preferably, the solubilizer is PEG40-hydrogenated castor-oil.

In one embodiment, the composition of the present invention comprises one or more thickener. Thickeners are used to give the shampoo a desirable consistency and improve its ability to spread and adhere to the hair. Common thickeners include cellulose derivatives like hydroxyethyl cellulose, guar gum, xanthan gum, polyacrylic acid thickeners.

In one embodiment, the composition of the present invention comprises one or more preservatives. Preservatives are added to shampoos to prevent the growth of microorganisms and extend the shelf life of the product. Preservatives may be added to the composition in an amount of 0.1 to 1.0 wt.-%.

Examples of preservatives used in shampoos include sodium benzoate, phenoxyethanol, 4,4'-dichloro 2-hydroxydiphenylether, potassium sorbate, benzyl alcohol, caprylyl glycol, ethylhexylglycerin, DMDM hydantoin, parabens (such as methylparaben, propylparaben, butylparaben), sorbic acid, and combinations thereof. Preferably, th composition comprises a compound selected from the group consisting of sodium benzoate, phenoxyethanol, 4,4'-dichloro 2-hydroxydiphenylether, potassium sorbate, benzyl alcohol, caprylyl glycol, ethylhexylglycerin, DMDM hydantoin, parabens (such as methylparaben, propylparaben, butylparaben), sorbic acid, and combinations thereof. Most preferred, the preservative is sodium benzoate.

In one embodiment, the composition of the present invention comprises one or more pH adjusters. pH adjusters are used to maintain the desired pH level of the shampoo, which is preferably slightly acidic to help keep the hair cuticles smooth.

Preferably, the composition comprises pH-adjusters in an amount of 0.1 to 0.8 wt.-%.

Common pH adjusters include citric acid, triethanolamine, sodium hydroxide, actic acid, phosphoric acid, and acetic acid. Preferably, the pH-adjuster is citric acid.

In one embodiment, the composition of the present invention comprises one or more fragrances. Fragrances are added to give the shampoo a pleasant scent. They can be a combination of natural or synthetic compounds. Preferably, the composition comprises a total amount of 0.1 to 1.5 wt.-% of fragrances.

In one embodiment, the composition of the present invention comprises one or more botanical extracts. Many shampoos contain botanical extracts that provide nourishing and conditioning benefits to the hair. Examples include aloe vera, chamomile, green tea, and lavender extracts.

In one embodiment, the composition of the present invention comprises one or more antioxidants. Antioxidants are sometimes included in shampoos to protect the hair from damage caused by free radicals. Common antioxidants used in shampoos include vitamin E, vitamin C, and various plant extracts.

In one embodiment, the composition of the present invention comprises one or more humectants. Humectants help to retain moisture in the hair and prevent it from becoming dry and brittle. Common humectants used in shampoos include glycerin, propylene glycol, and sorbitol.

In one embodiment, the composition of the present invention comprises one or more UV-Filters to protect the human hair from UV light, leading to decoloration of the hair and protecting the product from colour-fading.

The composition of the present invention can also comprise a builder (herein also called "chelating agent"). In one embodiment, the composition of the invention comprises at least one builder selected from non-phosphate-based builders such as sodium gluconate, citrate(s), silicate(s), carbonate(s), phosphonate(s), amino carboxylate(s), polycarboxylate(s), polysulfonate(s), and polyphosphonate(s). In one embodiment, compositions of the current invention are essentially devoid of phosphate-based builders. In another preferred embodiment, the composition comprises a phosphonate, wherein the phosphonate is preferably DTPMP and/or HEDP. In one embodiment, the compositions of the invention comprise at least one "citrate" selected from the mono- and the dialkali metal salts and in particular the mono- and preferably the trisodium salt of citric acid, ammonium or substituted ammonium salts of citric acid as well as citric acid as such. Citrate can be used as the anhydrous compound or as the hydrate, for example as sodium citrate dihydrate. In one embodiment, the builder is a sequestering builder. In one embodiment, the builder is a strong sequestering builder. Strong sequestering builders include, but are not limited to, Ethylenediaminetetraacetic acid (EDTA), Ethylene diamine tetra(methylene phosphonic acid (EDTMP), Nitrilo trimethylene phosphonic acid (NTMP), Diethylenetriamine Penta(Methylene Phosphonic acid) (DTPMP), methylgly-cinediacetic acid (MGDA), Nitrilotriacetic acid (NTA), 1-Hydroxy Ethylidene-1,1-Diphosphonic acid (HEDP), sodium tripolyphosphate (STPP), iminodisuccinic acid (IDS), N,N-diacetic acid tetra sodium salt (GLDA), pyrophosphate, ethylenediaminedisuccinic acid (EDDS), and combinations thereof. Preferably, the composition comprises MGDA and/or EDDS.

Preferably, one or more, preferably all, builders in the composition are biobased and/or biodegradable. Most preferably, one or more, preferably all, builders in the composition are biobased and biodegradable. A biodegradable builder is readily biodegradable in an aerobic aqueous medium according to the OECD guideline 301. Biodegradable complexing agents include but are not limited to, EDDS, MGDA, GLDA, and citrate.

In one embodiment, the composition of the present invention comprises a protease stabilizing system. Preferably, the protease stabilizing system comprises at least one compound selected from the group consisting of polyols (preferably, 1,3-propanediol, ethylene glycol, glycerol, 1,2-propanediol, or sorbitol), inorganic salts (preferably, CaCl2, and MgCl2), short chain (preferably, C1-C3) carboxylic acids or salts thereof (preferably, formic acid, formate (preferably, sodium formate), acetic acid, acetate, or lactate), borate, boric acid, boronic acids (preferably, 4-formyl phenyl-boronic acid (4-FPBA)), peptide aldehydes (preferably, Benzyloxycarbony-VAL-H (Z-VAL-H or Cbz-VAL-H) or Ben-zyloxycarbony-GAY-H (Z-GAY-H or Cbz-GAY-H), peptide acetals, and peptide aldehyde hydrosulfite adducts. Preferably, the protease stabilizing system comprises a combination of at least two of the compounds selected from the group consisting of salts, polyols, and short chain carboxylic acids and preferably one or more of the com-pounds selected from the group consisting of borate, boric acid, boronic acids (preferably, 4-formyl phenylboronic acid (4-FPBA)), peptide aldehydes, peptide acetals, and peptide aldehyde hydrosulfite adducts. In a particularly preferred embodiment, the stabilizing system comprises a protease inhibitor, preferably selected from borate, boric acid, boronic acids (preferably, 4-FPBA), peptide aldehydes (preferably, peptide aldehydes like Z-VAL-H or Z-GAY-H), peptide acetals, and peptide aldehyde hydrosulfite adducts, preferably the protease inhibitor is a peptide aldehyde, preferably Z-VAL-H or Z-GAY-H. In one embodiment, the stabilizing system does not comprise a protease inhibitor. Preferably, the composition is boron-free. Preferably, the protease formulation comprises a calcium salt, preferably calcium chloride.

In addition, solid hair-care compositions may also contain one or more fillers. Preferred fillers are selected from the group consisting of starch, dextrose, kaolin clay, bentonite clay, rhassoul clay, arrowroot powder, rice flour, and oat flour. Most preferred fillers are selected from starch, dextrose, and combinations thereof. These fillers help to provide structure and stability to the solid hair-care compositions, as well as to enhance its cleansing properties. The use of fillers in solid hair-care compositions can also help to reduce costs and improve the overall performance of the product.

It is important to note that the above-cited list of ingredients is not exhaustive, and additional ingredients may be included based on specific formulation requirements or desired product claims. The proportions and combinations of these ingredients can vary depending on the manufacturer's proprietary formulation.

### Conditioning agent

Preferably, the composition of the present invention is essentially devoid of alkyl dimethyl polysiloxanes. More preferably, the composition is essentially devoid of dimethyl polysiloxanes. Even more preferred, the composition is essentially devoid of polymeric siloxanes, in particular dimethyl siloxanes. Preferably, the composition is essentially devoid of quaternary ammonium compounds, in particular polyquaternium polymers. Preferably, the composition is essentially devoid of quaternary ammonium compounds, monomeric or polymeric, having the monomer formula [R1R2R3R4N]+X- or R1R2N+(R3)(R4)X-, wherein X- is an anion, preferably chloride (CI-), bromide (Br-), iodide (I-), sulfate (SO42-), or any other suitable anion and R1, R2, R3, and R4 represent organic groups independently selected from each other, preferably alkyl groups with C14-C22, preferably C16-C22 carbon atoms, which can be saturated or unsaturated. In case of polyquaternium polymers any of R1, R2, R3, or R4 can comprise alkyl groups linking the nitrogen atoms.

Thus, even further preferred, the composition is essentially devoid of polymeric siloxanes, in particular dimethyl siloxanes, and quaternary ammonium compounds, in particular polyquaternium polymers.

Preferably, the composition of the present invention does not comprise tertiary ammonium compounds ("pseudoquats). Preferably, the composition does not comprise quaternary ammonium compounds and tertiary ammonium compounds.

Further preferred, the composition is essentially devoid of a synthetic conditioning polymer. Most preferably, the composition is essentially devoid of a synthetic conditioning agent.

Preferably, the composition of the present invention does not comprise alkyl dimethyl polysiloxanes. More preferably, the composition does not comprise dimethyl polysiloxanes. Even more preferred, the composition does not comprise polymeric siloxanes, in particular dimethyl siloxanes, preferably does not comprise polymeric siloxanes and quaternary ammonium compounds, in particular polyquaternium polymers. Further preferred, the composition does not comprise synthetic conditioning polymer. Most preferably, the composition does not comprise synthetic conditioning agent.

Most preferably the composition does not comprise a polyquaternium ammonium compounds, preferably polyquaternium polymers, and/or a polymeric siloxane and/or siloxan-derivatives (e.g. amodimethicone). Preferably, the composition does not comprise any of dimethicone, cyclomethicone, polyquaternium-7, polyquaternium-10, behentrimonium chloride, quaternium-80, cetrimonium chloride, and quaternium-18. More preferably, the composition does not comprise alkyl dimethyl polysiloxanes. Most preferably, the composition does not comprise dimethyl polysiloxanes.

However, to further support the conditioning effect provided by the protease in the hair-care composition of the present invention, the composition may comprise a certain amount of non-synthetic conditioning agents. Thus, in one embodiment, the hair-care composition comprises one or more non-synthetic conditioning agent.

Preferably, in this embodiment, the hair-care composition comprises one or more non-synthetic conditioning agent in total amount of 0.1 to 10 wt.-%, preferably 1 to 5 wt.-%.

Preferably, the non-synthetic conditioning agent is biodegradable. A biodegradable conditioning agent is readily biodegradable in an aerobic aqueous medium according to the OECD guideline 301.

Preferred non-synthetic conditioning agents are naturally derived compounds, natural-derived oils or natural-derived waxes. Waxes are preferably added to the formulation in the form of wax dispersions. Non-synthetic conditioning agents are preferably selected from the group consisting of sunflower oil, coconut oil, argan oil, shea butter, jojoba oil, panthenol, avocado oil, candelilla wax, soy wax, honey, silk protein, and mixtures thereof. Most preferred, the compositions composition comprises one or more non-synthetic conditioning agents selected from the group consisting of jojoba oil, shea butter, sunflower oil, rape seed oil, hardened rape seed oil and ethylene glycol distearate.

In one embodiment, the composition of the present invention is essentially devoid of any conditioning agent. In another embodiment, the composition does not comprise any conditioning agent.

### Preferred compositions

A typical hair-care composition comprises various ingredients that work together to cleanse and condition the hair. While the specific composition may vary depending on the desired properties and target audience of the hair-care composition, the following the preferred ingredients:
1. Anionic Surfactants: Anionic surfactants, such as alkylethersulfates, are the primary cleansing agents in shampoos. They help to remove dirt, oil, and other impurities from the hair and skin.
2. Amphoteric Surfactants: Amphoteric surfactants, such as betaines, are used in shampoos to provide mild cleansing and conditioning properties. They also help to improve foam stability and reduce skin irritation.
3. Non-ionic Surfactants: Non-ionic surfactants, such as alkylpolyglucosides, are used in shampoos to provide additional cleansing and foaming properties. They are typically mild and gentle on the hair and skin. Non-ionic surfactants also frequently used in shampoo compositions are tween-type Surfactants / Sorbitane. Tween-type surfactants, such as polysorbate, are used in shampoos as emulsifiers and solubilizers. They help to improve the solubility of other ingredients in the formulation and enhance the overall performance of the shampoo.
4. Salts: Some shampoo compositions may also include salts. Salts, such as sodium chloride, can act as viscosity modifiers, improving the texture and spreadability of the shampoo. Additionally, salts like magnesium sulfate may be added to provide benefits for skin health and to soothe an irritated skin.

Thus, in one embodiment, the composition of the present invention, preferably hair-care composition, more preferably rinse-off composition, even more preferably shampoo composition, comprises
a) one or more proteases, preferably, wherein the protease has at least 80%, at least 85%, at least 90%, at least 95%, at least 96%, at least 97%, at least 98%, or at least 99%, but less than 100% sequence identity to SEQ ID NO: 1 and preferably comprises compared to SEQ ID NO: 1 the amino acid substitution R101D or R101E, preferably R101E, according to the numbering of SEQ ID NO: 2,
b) one or more surfactants, with preferably at least one of the surfactants selected from the group consisting of
   a. ionic surfactant, preferably anionic surfactant,
   b. amphoteric surfactant, and
   c. non-ionic surfactant,
c) preferably one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In one embodiment, the composition of the present invention, preferably hair-care composition, more preferably rinse-off composition, even more preferably shampoo composition, comprises
a) one or more proteases,
b) one or more surfactants, with preferably at least one of the surfactants selected from the group consisting of
   a. ionic surfactant, preferably anionic surfactant,
   b. amphoteric surfactant, and
   c. non-ionic surfactant,
c) preferably one or more salt,
d) one or more non-synthetic conditioning agents,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In another embodiment, the present invention is directed to a hair-care composition comprising
a) one or more proteases,
b) one or more surfactants, with preferably at least one of the surfactants selected from the group consisting of
   a. ionic surfactant,
   b. amphoteric surfactant, and
   c. non-ionic surfactant,
c) preferably one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent, preferably essentially devoid of any conditioning agent, and
   wherein the pH of the composition is pH 4.0-8.5.

In one embodiment, the present invention is directed to a
liquid hair-care composition, preferably rinse-off composition, more preferably shampoo composition, comprising
a) 0.001 to 1.5 wt.-% total amount of one or more proteases,
b) 5 to 30% wt.-% total amount one or more surfactants, with preferably at least one of the surfactants selected from the group consisting of
   a. 0 to 30 wt.-% ionic surfactant,
   b. 0 to 30 wt.-% amphoteric surfactant, and
   c. 0 to 30 wt.-% non-ionic surfactant,
c) preferably 0.1 to 5 wt.-% of one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In one embodiment, the present invention is directed to a
liquid hair-care composition, preferably rinse-off composition, more preferably shampoo composition, comprising
a) 0.001 to 1.5 wt.-% total amount of one or more proteases,
b) 5 to 30% wt.-% total amount one or more surfactants, with preferably at least one of the surfactants selected from the group consisting of
   a. 0 to 30 wt.-% ionic surfactant,
   b. 0 to 30 wt.-% amphoteric surfactant, and
   c. 0 to 30 wt.-% non-ionic surfactant,
c) preferably 0.1 to 5 wt.-% of one or more salt,
d) 0.1 to 10 wt.-% total amount of one or more non-synthetic conditioning agents,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

Preferably, the present invention is directed to a
liquid hair-care composition, preferably rinse-off composition, more preferably shampoo composition, comprising
a) 0.001 to 1.5 wt.-% total amount of one or more proteases,
b) 15 to 30 wt.-% total amount one or more surfactants, with preferably at least one of the surfactants selected from the group consisting of
   a. 1 to 30 wt.-% ionic surfactant,
   b. 1 to 30 wt.-% amphoteric surfactant, and
   c. optionally 1 to 30 wt.-% non-ionic surfactant,
c) preferably 0.1 to 5 wt.-% of one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In a particularly preferred embodiment, the present invention is directed to a
liquid hair-care composition, preferably rinse-off composition, more preferably shampoo composition, comprising
a) 0.001 to 1.5 wt.-% total amount of one or more proteases,
b) 15 to 30 wt.-% total amount one or more surfactants, with preferably at least one of the surfactants selected from the group consisting of
   a. 10-20 wt.-% ionic surfactant, preferably an anionic surfactant, preferably an alkylethersulfate, most preferably sodium laurylethersulfate,
   b. 5-10 wt.-% amphoteric surfactant, preferably a betaine, preferably an alkyl dimethyl betaine, a sulfo betaine, and combinations thereof, most preferably cocoamidoproylbetaine), and
   c. preferably 0 wt.-% non-ionic surfactant,
c) 0.1 to 5 wt.-% of one or more salt, preferably a chloride-containing salt, preferably sodium chloride,
d) optionally, 0.1 to 1.0 wt.-% of a preservative, preferably sodium benzoate,
e) optionally, 0.1 to 1.5 wt.-% of fragrances,
f) optionally, 0.1 to 0.8 wt.-% of a pH adjuster, preferably citric acid,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5, preferably pH 4.0-6.5.

In a particularly preferred embodiment, the present invention is directed to a
liquid hair-care composition, preferably rinse-off composition, more preferably shampoo composition, that consists of
a) 0.001 to 1.5 wt.-% total amount of one or more proteases,
b) 15 to 30 wt.-% total amount one or more surfactants, with preferably at least one of the surfactants selected from the group consisting of
   a. 10-20 wt.-% ionic surfactant, preferably an anionic surfactant, preferably an alkylethersulfate, most preferably sodium laurylethersulfate,
   b. 5-10 wt.-% amphoteric surfactant, preferably a betaine, preferably an alkyl dimethyl betaine, a sulfobetaine, and combinations thereof, most preferably cocoamidopropylbetaine), and
c) 0.1 to 5 wt.-% of one or more salt, preferably a chloride-containing salt, preferably sodium chloride,
d) 0.1 to 1.0 wt.-% of a preservative, preferably sodium benzoate,
e) 0.1 to 1.5 wt.-% of fragrances,
f) 0.1 to 0.8 wt.-% of a pH adjuster, preferably citric acid, and
g) up to 100 wt.-% of water, and
   wherein the pH of the composition is pH 4.0-8.5, preferably pH 4.0-6.5.

In a further preferred embodiment, the present invention is directed to a
liquid hair-care composition, preferably rinse-off composition, more preferably shampoo composition, that consists of
a) 0.001 to 1.5 wt.-% total amount of one or more proteases,
b) 15 to 30 wt.-% total amount one or more surfactants, with preferably at least one of the surfactants selected from the group consisting of
   a. 10-20 wt.-% ionic surfactant, preferably an anionic surfactant, preferably an alkylethersulfate, most preferably sodium laurylethersulfate,
   b. 5-10 wt.-% amphoteric surfactant, preferably a betaine, preferably an alkyl dimethyl betaine, a sulfo betaine, and combinations thereof, most preferably cocoamidoproylbetaine), and
c) 0.1 to 5 wt.-% of one or more salt, preferably a chloride-containing salt, preferably sodium chloride,
d) 0.1 to 10 wt.-% total amount of one or more non-synthetic conditioning agents,
e) 0.1 to 1.0 wt.-% of a preservative, preferably sodium benzoate,
f) 0.1 to 1.5 wt.-% of fragrances,
g) 0.1 to 0.8 wt.-% of a pH adjuster, preferably citric acid, and
h) up to 100 wt.-% of water, and
   wherein the pH of the composition is pH 4.0-8.5, preferably pH 4.0-6.5.

In another embodiment, the present invention is directed to a
solid hair-care composition, preferably rinse-off composition, more preferably shampoo composition, comprising
a) 0.001 to 1.5 wt.-% total amount of one or more proteases,
b) 35 to 95 wt.-% total amount one or more surfactants, with preferably at least one of the surfactants selected from the group consisting of
   a. 0 to 95 wt.-% ionic surfactant,
   b. 0 to 95 wt.-% amphoteric surfactant, and
   c. 0 to 95 wt.-% non-ionic surfactant,
c) preferably 0.1 to 5 wt.-% of one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In another embodiment, the present invention is directed to a
solid hair-care composition, preferably rinse-off composition, more preferably shampoo composition, comprising
a) 0.001 to 1.5 wt.-% total amount of one or more proteases,
b) 35 to 95 wt.-% total amount one or more surfactants, with preferably at least one of the surfactants selected from the group consisting of
   a. 0 to 95 wt.-% ionic surfactant,
   b. 0 to 95 wt.-% amphoteric surfactant, and
   c. 0 to 95 wt.-% non-ionic surfactant,
c) preferably 0.1 to 5 wt.-% of one or more salt,
d) of 0.1 to 10 wt.-% total amount of one or more non-synthetic conditioning agents,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

Preferably, the present invention is directed to a
solid hair-care composition, preferably rinse-off composition, more preferably shampoo composition, comprising
a) 0.001 to 1.5 wt.-% total amount of one or more proteases,
b) 35 to 95 wt.-% total amount one or more surfactants, with preferably at least one of the surfactants selected from the group consisting of
   a. 15 to 95 wt.-% ionic surfactant,
   b. 10 to 95 wt.-% amphoteric surfactant, and
   c. 10 to 95 wt.-% non-ionic surfactant,
c) preferably 0.1 to 5 wt.-% of one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In one embodiment, the present invention is directed to a
hair-care composition, preferably rinse-off composition, more preferably shampoo composition, comprising
a) 0.001 to 1.5 wt.-% total amount of one or more proteases, and
   when being in liquid form
b) 5 to 30% wt.-% total amount one or more surfactants, with preferably at least one of the surfactants selected from the group consisting of
   a. 0 to 30 wt.-% ionic surfactant,
   b. 0 to 30 wt.-% amphoteric surfactant, and
   c. 0 to 30 wt.-% non-ionic surfactant,
   or when being in solid form
b) 35 to 95 wt.-% total amount one or more surfactants, with preferably at least one of the surfactants selected from the group consisting of
   a. 0 to 95 wt.-% ionic surfactant,
   b. 0 to 95 wt.-% amphoteric surfactant, and
   c. 0 to 95 wt.-% non-ionic surfactant,
c) preferably 0.1 to 5 wt.-% of one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5.

In one embodiment, the composition is essentially devoid of one or more of the compounds selected from the group consisting of sulfate, phosphate, and parabens.

In one embodiment, the composition is essentially devoid of sulfate.

In one embodiment, the composition is essentially devoid of phosphate.

In another embodiment, the composition is essentially devoid of parabens.

In one embodiment, the composition does not comprise one or more of the compounds selected from the group consisting of sulfate, phosphate, and parabens. In a preferred embodiment, the composition does not comprise sulfate, phosphate, and parabens.

### Methods and use

The present invention is also directed to the use of the composition described herein, preferably a composition comprising
a) one or more proteases,
b) one or more surfactants, with preferably at least one of the surfactants selected from the group consisting of
   a. ionic surfactant,
   b. amphoteric surfactant, and
   c. non-ionic surfactant,
c) preferably one or more salt,
   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
   wherein the pH of the composition is pH 4.0-8.5,
   as a hair-care composition, preferably as a rinse-off composition, most preferably as shampoo composition.

The composition of the present invention can be used for human or animal hair, preferably for human hair.

The present inventors have revealed that the composition of the present invention smoothens the hair surface, in particular the hair cuticle. The present inventors have revealed that the composition of the present invention improved combability of the hair, preferably wet and/or dry combability, most preferably dry combability, particularly without the addition of compounds that remain on the hair after rinsing-off the composition, preferably without the addition of alkyl dimethyl polysiloxanes, more preferably without the addition of dimethyl polysiloxanes, even more preferably without the addition of polymeric siloxanes, preferably without the addition of polymeric siloxanes and quaternary ammonium compounds, preferably polyquaternium polymers, further preferred, without the addition of a synthetic conditioning agent, preferably without the addition of any conditioning agent.

Thus, the present invention allows for smoothening the hair surface without depositing a compound on the surface of the hair, preferably without depositing alkyl dimethyl polysiloxanes, more preferably without depositing dimethyl polysiloxanes, even more preferably without depositing polymeric siloxanes, preferably without depositing polymeric siloxanes and quaternary ammonium compounds, preferably polyquaternium polymers, further preferred, without depositing a synthetic conditioning agent, preferably without depositing any conditioning agent on the surface of the hair.

Thus, in one embodiment, the present invention is directed to a method for improving the combability of hair, preferably the dry combability, comprising the steps of
i. contacting hair with a composition of the present invention under conditions suitable for the protease of the composition to exert proteolytic activity on the hair, preferably the hair surface, preferably the hair cuticle, and
ii. rinsing-off the composition from the hair; and
iii. optionally drying the hair.

Thus, in one embodiment, the present invention is directed to a method for improving the combability of hair, preferably the dry combability, comprising the steps of
i. contacting hair with a composition, preferably hair-care composition, more preferably rinse-off composition, even more preferably shampoo composition, comprising
   a) one or more proteases, preferably, wherein the protease has at least 80%, at least 85%, at least 90%, at least 95%, at least 96%, at least 97%, at least 98%, or at least 99%, but less than 100% sequence identity to SEQ ID NO: 1 and preferably comprises compared to SEQ ID NO: 1 the amino acid substitution R101D or R101E, preferably R101E, according to the numbering of SEQ ID NO: 2,
   b) one or more surfactants, with preferably at least one of the surfactants selected from the group consisting of
      a. ionic surfactant, preferably anionic surfactant,
      b. amphoteric surfactant, and
      c. non-ionic surfactant,
   c) preferably one or more salt,
      wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent and
      wherein the pH of the composition is pH 4.0-8.5, under conditions suitable for the protease of the composition to exert proteolytic activity on the hair, preferably the hair surface, preferably the hair cuticle, and
ii. rinsing-off the composition from the hair; and
iii. optionally drying the hair.

Thus, in one embodiment, the present invention is directed to a method for smoothening the hair surface, preferably the hair cuticle, comprising the steps of
i. contacting hair with a composition of the present invention under conditions suitable for the protease of the composition to exert proteolytic activity on the hair, preferably the hair surface, preferably the hair cuticle, and
ii. rinsing-off the composition from the hair; and
iii. optionally drying the hair.

Thus, in one embodiment, the present invention is directed to the use of a composition of the present invention for improving the combability of hair, preferably the dry combability.

Thus, in one embodiment, the present invention is directed to the use of a composition of the present invention for smoothening the hair surface, preferably the hair cuticle.

In one particular embodiment, the composition of the present invention is used in combination with a synthetic conditioner in order to accommodate that the conditioning effect of the composition of the present invention might be less pronounced after first treatment of the hair compared to a traditional composition comprising no protease. Thus, in this embodiment, the treatment of the hair is accomplished in two steps, wherein the first step is the treatment of the hair with the composition of the present invention, preferably as a rinse-off composition, and wherein the second step is a treatment of the hair with a composition, preferably a leave-in composition, comprising a conditioning agent, preferably a synthetic conditioning agent, wherein the second step is performed compared to the first step in a lower number. Thus, the present invention is directed to a method for improving the combability of hair, preferably the dry combability, or for smoothening the hair surface comprising the steps of
i. contacting hair with a composition of the present invention under conditions suitable for the protease of the composition to exert proteolytic activity on the hair, preferably the hair surface, preferably the hair cuticle and
ii. rinsing-off the composition of step i) from the hair;
iii. optionally drying the hair;
iv. thereafter contacting the hair with a composition comprising a conditioning agent, preferably a synthetic conditioning agent and devoid of a protease,
v. optionally rinsing-off the composition from step iv) from the hair, wherein at least parts of the conditioning agent of the composition of step iv remains on the hair;
vi. optionally drying the hair;
vii. repeating steps i. to vi. 1 to 20 times, preferably 5 to 10 times, and then
viii. repeating only steps i. to iii. for 1 to 30 times, preferably 5 to 20 times.

Thus, the present invention is directed to a method for improving the combability of hair, preferably the dry combability, or for smoothening the hair surface comprising the steps of
i. contacting hair with a first composition, preferably hair-care composition, more preferably rinse-off composition, even more preferably shampoo composition, comprising
   a) one or more proteases, preferably, wherein the protease has at least 80%, at least 85%, at least 90%, at least 95%, at least 96%, at least 97%, at least 98%, or at least 99%, but less than 100% sequence identity to SEQ ID NO: 1 and preferably comprises compared to SEQ ID NO: 1 the amino acid substitution R101D or R101E, preferably R101E, according to the numbering of SEQ ID NO: 2,
   b) one or more surfactants, with preferably at least one of the surfactants selected from the group consisting of
      a. ionic surfactant, preferably anionic surfactant,
      b. amphoteric surfactant, and
      c. non-ionic surfactant,
   c) preferably one or more salt, and

   wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent, even further preferred is essentially devoid of any conditioning agent, and
   wherein the pH of the composition is pH 4.0-8.5,
   under conditions suitable for the protease of the composition to exert proteolytic activity on the hair, preferably the hair surface, preferably the hair cuticle, and
ii. rinsing-off the composition of step i) from the hair;
iii. optionally drying the hair;
iv. contacting the hair with a second composition comprising a conditioning agent, preferably a synthetic conditioning agent and devoid of a protease,
v. optionally rinsing-off the composition from step iv) from the hair, wherein at least parts of the conditioning agent of the composition of step iv remains on the hair;
vi. optionally drying the hair;
vii. repeating steps i. to vi. 1 to 20 times, preferably 5 to 10 times, and then
viii. repeating only steps i. to iii. for 1 to 30 times, preferably 5 to 20 times.

For such an embodiment, the present invention is also directed to a kit. Therefore, in one embodiment, the present invention is directed to a kit for performing the method as described above. Thus, in one embodiment, the present invention is directed to a kit comprising
a) a first composition of the present invention in an amount suitable to be used in 1 to 30, preferably 15 to 30, methods of hair-treatment; and
b) a second composition comprising a conditioning agent, preferably a synthetic conditioning agent, and being devoid of a protease in an amount suitable to be used in 1 to 20, preferably 5 to 10, methods of hair-treatment.

Thus, in one embodiment, the present invention is directed to a kit comprising
a) a composition, preferably hair-care composition, more preferably rinse-off composition, even more preferably shampoo composition, comprising
   i) one or more proteases, preferably, wherein the protease has at least 80%, at least 85%, at least 90%, at least 95%, at least 96%, at least 97%, at least 98%, or at least 99%, but less than 100% sequence identity to SEQ ID NO: 1 and preferably comprises compared to SEQ ID NO: 1 the amino acid substitution R101D or R101E, preferably R101E, according to the numbering of SEQ ID NO: 2,
   ii) one or more surfactants, with preferably at least one of the surfactants selected from the group consisting of
      a. ionic surfactant, preferably anionic surfactant,
      b. amphoteric surfactant, and
      c. non-ionic surfactant, and
   iii) preferably one or more salt,
      wherein the composition is essentially devoid of alkyl dimethyl polysiloxanes, more preferably essentially devoid of dimethyl polysiloxanes, even more preferably essentially devoid of polymeric siloxanes, preferably essentially devoid of polymeric siloxanes and quaternary ammonium compounds, preferably polyquaternium polymers, further preferred, essentially devoid of a synthetic conditioning agent, even further preferred is essentially devoid of any conditioning agent, and
      wherein the pH of the composition is pH 4.0-8.5,
      in an amount suitable to be used in 1 to 30, preferably 15 to 30, methods of hair-treatment; and
c) a composition, preferably hair-care composition, more preferably leave-in composition, even more preferably shampoo composition, comprising a conditioning agent, preferably a synthetic conditioning agent, and being devoid of a protease,
   in an amount suitable to be used in 1 to 20, preferably 5 to 10, methods of hair-treatment.

### Examples

### Example 1

### Experimental Method

### Preparation of Hair Strands

### Dry Combing test

The hair strands (Caucasian hair, 15 cm/2 g, International Hair Importers & Products, USA, batch# 11) were cleansed by incubation in 6 % sodium lauryl ether sulfate (active matter), pH 6.5, for 30 minutes, followed by rinsing and submerging three times in water for two minutes each. Then the hair was bleached with 5 % hydrogen peroxide (pH 9.4) for 15 minutes, followed by two further cleansing steps.

Finally, all the hair strands were dried for 45 minutes hanging in a flow of warm air (approximately 55 °C).

All the preparations described were done by an automated system.

### Measurement of Combing Work

The hair strands were measured before and after treatment.

### Dry Combing

The hair was rinsed off for one minute using the automated rinsing and combing machine (1 l/min, 38 °C) and equilibrated for 16 hours under climate-controlled conditions (30 °C, 40% relative humidity). The hair strands were combed 23 times by a robotic system. The combing forces for the dry hair strands were recorded. Finally, the strands were deionized (reference measurements).

### Treatment

After the reference measurements, the hair strands were treated twice with the hair-care composition (0.25 g product per 1 g hair, incubation for 5 minutes). The formulation was rinsed-off for one minute using the automated rinsing and combing machine (1 l/min, 38 °C).

### Measurement of Combing Work, after Treatment

### Dry Combing

The hair was equilibrated for 16 hours under climate-controlled conditions (30 °C, 40 % relative humidity). The treated hair was then measured as described above.

### Evaluation of Residual Combing Work

For each formulation, the mean combing work of the ten tested strands is calculated from the integrated force values of the 4^{th} to the 23^{rd} combing stroke in each case.

### Calculation

The residual combing work is calculated as follows:
- Residual combing work [%] = (combing work after treatment/combing work before treatment) x 100%

### Test of Significance

Statistical significance (p< = 0.05) of differences before and after treatment was calculated using a paired t.test and is indicated by an asterisk (*) in tables of the combing results.

Statistical significance (p< = 0.05) of differences between test formulations was calculated using Tukey's test.

Tukey's test is a single-step multiple comparison procedure and statistical test used in conjunction with an ANOVA (analysis of variance) to find which means are significantly different from one another. If the ANOVA test indicates significant differences, a Tukey's test is performed. It compares all possible pairs of means and is based on a studentized range distribution (this distribution is similar to the distribution of t from the t.test). The test compares the means of every treatment to the means of every other treatment. It is applied simultaneously to the set of all pair wise comparisons and identifies where the difference between two means is greater than the standard error would be expected to allow. The threshold of the test is the HSD-range (Honestly Significant Difference). The HSD-range is determined by the signal-to-noise ratio, and the number of measurements. The difference between two samples has to be greater than the calculated HSD value to be significantly different at a 95% probability.

The following hair-care composition was used.

| | **Composition with Protease [wt%]** | **Composition without Protease [wt%]** |
|---|---|---|
| **Cocamidopropyl Betaine (Dehyton PK45, 45%)** | 5.4 | 5.4 |
| **Sodium Laureth Sulfate (Texapon N70, 70%)** | 14.3 | 14.3 |
| **Perfume** | 0.3 | 0.3 |
| **Na-Benzoat** | 0.5 | 0.5 |
| **Sodium chloride** | 1.6 | 1.6 |
| **Citric acid** | 0.3 | 0.3 |
| **Protease*** | 0.0015 | 0 |
| **Aqua dest.** | add 100 | add 100 |
| **pH** | 4.9 | 4.9 |
| **Appearance** | transparent | transparent |

| | | |
|---|---|---|
| *) Protease: SEQ ID NO:22 as described in EP 1921147 (SEQ ID NO: 1 of the present invention) with an amino acid glutamic acid (E) at position 101 (according to the BPN' numbering, i.e., according to the numbering of SEQ ID NO: 2 of the present invention). | | |

### Result

The result of this study is shown in Table 1.

**Table 1**

| | Mean Residual Combing Work [%] | SD residual [%] |
|---|---|---|
| **Composition with Protease** | 70.4 | 6.1* |
| **Composition with Protease - 30 min** | 51.3 | 8.0* |
| **Composition without Protease** | 79.0 | 5.3* |
| **Composition without Protease - 30 min** | 68.9 | 5.7* |

| | | |
|---|---|---|
| *) t-test significant | | |

As can be derived from Table 1, using a protease in a hair-care composition significantly improves combability.

## Claims

1. Hair-care composition comprising
a) one or more proteases,
b) one or more surfactants, with preferably at least one of the surfactants selected from the group consisting of
a. ionic surfactant,
b. amphoteric surfactant, and
c. non-ionic surfactant,
c) preferably one or more salt,
wherein the composition is essentially devoid of alkyl dimethyl polysiloxanes and
wherein the pH of the composition is pH 4.0-8.5.

2. The composition of claim 1, wherein the composition is a rinse-off composition, preferably a shampoo composition.

3. The composition of any of the preceding claims, wherein the composition is essentially devoid of dimethyl polysiloxanes, preferably wherein the composition is essentially devoid of polymeric siloxanes, preferably essentially devoid of polymeric siloxanes and quaternary ammonium compounds, preferably polyquaternium polymers.

4. The composition of any of the preceding claims, wherein the composition is essentially devoid of synthetic conditioning agents.

5. The composition of any of the preceding claims, wherein the ionic surfactant is an anionic surfactant or a cationic surfactant, preferably an anionic surfactant, preferably an alkylethersulfate.

6. The composition of any of the preceding claims, wherein the amphoteric surfactant is a betaine.

7. The composition of any of the preceding claims, wherein the non-ionic surfactant is selected from the group consisting of alkylpolyglucoside (APG), a sorbitan-esters, sorbitan-derivative, and combinations thereof.

8. The composition of any of the preceding claims, wherein composition comprises a solubilizer, preferably PEG40-hydrogenated castor-oil.

9. The composition of any of the preceding claims, wherein the protease is a subtilisin protease.

10. The composition of any of the preceding claims, wherein the composition comprises a protease stabilizing system, wherein the protease stabilizing system preferably comprises at least one compound selected from the group consisting of polyols (preferably, 1,3-propanediol, ethylene glycol, glycerol, 1,2-propanediol, or sorbitol), inorganic salts (preferably, CaCl2, MgCl2, or NaCl), short chain (preferably, C1-C3) carboxylic acids and/or salts thereof (preferably, formic acid, formate (preferably, sodium formate), acetic acid, acetate, or lactate), borate, boric acid, boronic acids (preferably, 4-formyl phenylboronic acid (4-FPBA)), peptide aldehydes (preferably, Z-VAL-H or Z-GAY-H), peptide acetals, and peptide aldehyde hydrosulfite adducts, preferably peptide aldehydes (preferably, Z-VAL-H or Z-GAY-H).

11. Method for improving the combability of hair, preferably the dry combability, comprising the steps of
i. contacting hair with a composition according to any of claims 1-10 under conditions suitable for the protease of the composition to exert proteolytic activity on the hair, preferably the hair surface, and
ii. rinsing-off the composition from the hair; and
iii. optionally drying the hair.

12. Method for smoothening the hair surface, comprising the steps of
i. contacting hair with a composition according to any of claims 1-10 under conditions suitable for the protease of the composition to exert proteolytic activity on the hair, preferably the hair surface, and
ii. rinsing-off the composition from the hair; and
iii. optionally drying the hair.

13. Use of a composition according to any of claims 1-10 for improving the combability of hair, preferably the dry combability.

14. Use of a composition according to any of claims 1-10 for smoothening the hair surface.
